# EUROPEAN PATENT SPECIFICATION

(11) **EP 1 861 364 B1**
(45) Date of publication and mention of the grant of the patent: **11.05.2011**
(21) Application number: 06711127.8
(22) Date of filing: 14.03.2006
(51) Int. Cl.: C07D 207/32

(54) **PREPARATION OF AN ATORVASTATIN INTERMEDIATE USING A PAAL-KNORR CONDENSATION**
HERSTELLUNG EINES ATORVASTATIN-ZWISCHENPRODUKTS UNTER ANWENDUNG EINER PAAL-KNORR-KONDENSATION
PRÉPARATION D'UN INTERMÉDIAIRE DE L'ATORVASTATINE EN UTILISANT UNE CONDENSATION DE PAAL-KNORR

(30) Priority: 14.03.2005 IE 20050136
(43) Date of publication of application: 05.12.2007
(73) Proprietor: C.P. Pharmaceuticals International C.V., New York, NY 10017 (US)
(72) Inventor: DAVIES, Simon, Dun Laoghaire, County Dublin (IE); DENHOLM, Alastair, Alexander, Dun Laoghaire, County Dublin (IE); KELLEHER, Patrick, Dun Laoghaire, County Dublin (IE); TULLY, William, Eugene, Dun Laoghaire, County Dublin (IE)
(74) Representative: Rudge, Andrew John
(86) International application number: PCT/IE2006/000014
(87) International publication number: WO 2006/097909

(56) References cited:
- WO-A-02/43667
- WO-A-2004/046105
- US-A- 3 928 380
- US-A1- 2003 195 353
- WINTERS R T ET AL: "NONPEPTIDE ANGIOTENSIN II RECEPTOR ANTAGONISTS. 1. SYNTHESIS AND INVITRO STRUCTURE-ACTIVITY RELATIONSHIPS OF ((((1H-PYRROL-1-YLACETYL) AMINO)PHENYL)METHYL)IMIDAZOLE DERIVATIVES AS ANGIOTENSIN II RECEPTOR ANTAGONISTS" JOURNAL OF MEDICINAL CHEMISTRY, AMERICAN CHEMICAL SOCIETY. WASHINGTON, US, vol. 36, 1993, pages 1735-1745, XP000918877 ISSN: 0022-2623
- HAGMANN W K ET AL: "Substituted 2-aminopyridines as inhibitors of nitric oxide synthases" BIOORGANIC & MEDICINAL CHEMISTRY LETTERS, OXFORD, GB, vol. 10, no. 17, September 2000 (2000-09), pages 1975-1978, XP004214150 ISSN: 0960-894X
- BOWERS ET AL.: "Application of the 2,5-Dimethylpyrrole Group as a New and Orthogonal Amine-Protecting Group in Oligosaccharide Synthesis" JOURNAL OF ORGANIC CHEMISTRY, vol. 63, 1998, pages 4570-4571, XP002379014

## Description

The invention relates to an improved process for preparing an intermediate used in the process for the preparation of atorvastatin.

Atorvastatin, [R-R*,R*)]-2-(4-fluorophenyl-β,δ-dihydroxy-5-(1-methylethyl)-3-phenyl-4-[(phenylamino)carbonyl]-1H-pyrrole-1-heptanoic acid, the active pharmaceutical in Lipitor™, is pharmacologically active in humans and is useful as a hypolipidemic and hypocholesterolemic agent. In particular, atorvastatin is useful as a selective and competitive inhibitor of the enzyme 3-hydroxy-3-methylglutaryl-coenzyme A (HMG-CoA) reductase, the rate-limiting enzyme that converts 3-hydroxy-3-methylglutaryl-coenzyme A to mevalonate, a precursor of sterols such as cholesterol. The conversion of HMG-CoA to mevalonate is an early and rate-limiting step in cholesterol biosynthesis.

United States Patent Numbers 5,216,174; 5,245,047; 5,248,793; 5,397,792; 5,342,952; 5,298,627; 5,446,054; 5,470,981; 5,489,690; 5,489,691; 5,5109,488; WO97/03960; WO98/04543 and WO99/32434 disclose various processes and key intermediates for preparing atorvastatin.

One manufacturing process for atorvastatin includes a Paal-Knorr condensation reaction to form an acetonide ester at the key convergent step as described in US 5,280,126, US 6,476,235 and US 6,545,153. In this reaction the complete molecular framework is assembled in good yield. However the rate of formation of the acetonide ester is low.

Others (Szakal et al J Org Chem 1986, 51, 621-624; Amarnath et al: J Org Chem 1991, 56 6924-6931) have tried to determine the exact working mechanism of the Paal Knorr condensation reaction, however the mechanism is still not completely understood.

WO-2004/046105 describes a process for preparing atorvastatin involving a Paal-Knorr condensation catalysed by an organic acid

There is thus a need to optimise this rate limiting step in the preparation of atorvastatin.

### Statements of Invention

According to the invention there is provided a process for preparing a compound of formula 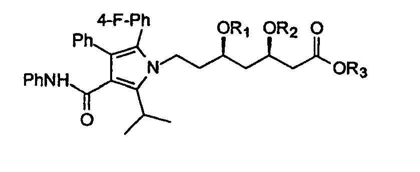 comprising reacting a 1,4-diketone of the formula 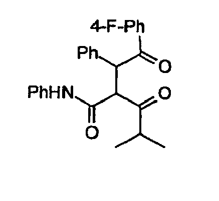 with a primary amine of the formula 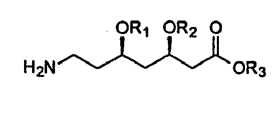 wherein R₁ and R₂ may be the same or different and are selected from any one or more of
H,
C₁-C₆ alkyl which may be straight or branched, substituted or unsubstituted with a halogen group,
C₁-C6 alkoxy group,
or R₁ and R₂ together represent an alkylidene group of the formula CRₐR_{b} wherein Rₐ and R_{b} may be the same or different and are selected from any one or more of a C₁-C₁₁ alkyl group,
and
R₃ is selected from any one or more of a C₁-C₆ alkyl group,
in the presence of a catalyst, the catalyst comprising a salt wherein the salt comprises a tertiary aromatic or aliphatic amine salt of an organic acid.

In one embodiment of the invention R₁ and R₂ together represent isopropylidene and R₃ is a tertiary butyl.

The primary amine may be 1,1-dimethyl-(4R-*cis*)-6-(2-aminoethyl)-2,2-dimethyl-1,3-dioxane-4-acetate.

In one embodiment of the invention the organic acid is a weak organic acid. The weak organic acid may have a pKa ≥ 2.

In one embodiment of the invention the weak organic acid is any carboxylic acid having a C₁-C₆ alkyl group which may be straight or branched. The weak organic acid may be selected from any one or more of pivalic acid, formic acid, acetic acid and isobutyric acid.

The tertiary amine may be selected from any one or more of triethylamine (Et₃N), diisopropylethyl amine (DIPEA), pyridine, 1-butylimidazole or n-ethylmorpholine.

In one embodiment of the invention the catalyst salt is selected from any one or more of pyridine.pivalate, n-ethylmorpholine.pivalate, triethylamine.acetate, triethylamine.formate, triethylamine.p-toluene sulphonate, triethyamine, triethylamine.isobutyrate, triethylamine.oxalate, triethylamine.triflouroacetate, 1-butylimidazole.pivalate, 1-butylimidazole.isobutyrate, 1-butylimidazole.oxalate, 1-butylimidazole.trifluroacetate.

In one embodiment of the invention the reaction is carried out in the presence of an inert organic solvent. The inert organic solvent may be selected from Hexane:THF, MTBE:THF, and toluene THF is tetrahydrofuran. MTBE is Methyltert.Butyl Ether.

In one embodiment of the invention the reaction is carried out at a pH of greater than about pH7.

One aspect of the invention provides that the water formed during the reaction is removed azeotropically.

In one embodiment of the invention the reaction is carried out at a temperature of between 40 and 120°C.

### Detailed Description

The invention relates to an improved catalyst for the acceleration of the critical Paal-Knorr reaction. Throughput is considerably improved over the prior art.

We have discovered an improved catalyst for the Paal-Knorr reaction to prepare the critical atorvastatin intermediate tert-butyl 2-((4R,6S)-6-((3-(phenylcarbamoyl)-5-(4-fluorophenyl)-2-isopropyl-4-phenyl-1H-pyrrol-1-yl)methyl)-2,2-dimethyl-1,3-dioxan-4-yl)acetate. It has been found that the addition of an amine to an organic acid currently used to catalyse the reaction, affords a dramatically shorter reaction time as well as an improved yield.

The invention will be more clearly understood from the following description thereof given by way of example only.

The present invention provides an improved process for preparing the acetonide ester of atorvastatin (tert-butyl 2-((4R,6S)-6-((3-(phenylcarbamoyl)-5-(4-fluorophenyl)-2-isopropyl-4-phenyl-1H-pyrrol-1-yl)methyl)-2,2-dimethyl-1,3-dioxan-4-yl)acetate). The acetonide ester is prepared with improved throughput with an increased reaction rate, high yield and a high level of purity.

The current process for preparing the acetonide ester of atorvastatin is carried out using a Paal Knorr reaction by reacting the diketone of atorvastatin, 4-fluoro- (2-methyl-1-oxopropyl-γ-oxo-*N-*β-diphenyl-benzenebutaneamide with the primary amine, 1,1-dimethyl-(4R-*cis*)-6-(2-aminoethyl)-2,2-dimethyl-1,3-dioxane-4-acetate. The reaction is carried out in the presence of an acid catalyst in an inert solvent. Full details of the reaction are given in US5,280,126, US6,476,235 and US6,545,153.

Neither the precise mechanism of the Paal-Knorr reaction nor the identity of the rate-determining step is known although studies have shed light on the presumed mechanistic pathway (Szakal et al, J Org Chem 1986,51,621-624). Scheme 1 below taken from Amarnath et al (J. Org. Chem. 1991, 56 6924-6931) shows a proposed mechanism of the reaction.

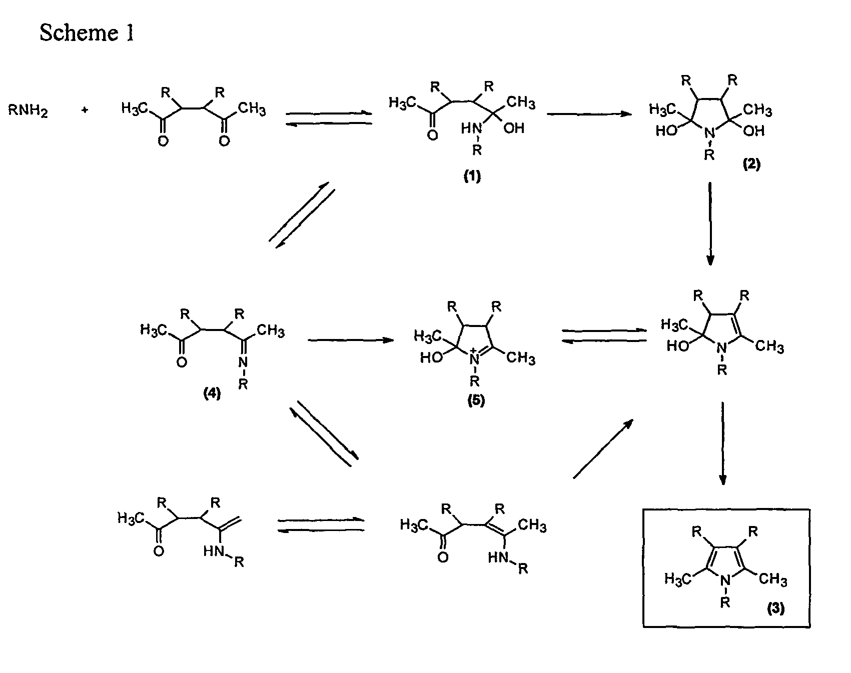

The proposed mechanism involves the addition of the amine component to one of the carbonyl carbons to form a hemiaminal (1). Intramolecular attack on the second carbonyl affords a diol (2), which must eliminate water (x2) to form a pyrrole (3).

Alternatively, attack at one carbonyl and elimination of water leads to the formation of an imine (4), which can then form a pyrrole (3) via enamines and water elimination.

Irrespective of the path chosen, the rate determining step appears to be the cyclisation to form either the diol (2) or the quaternary iminium salt (5). The Paal Knorr transformation is acid catalysed. In the case of Atorvastatin, the pyrrole ring formation of the acetonide ester is a key rate limiting step.

In the present invention it has been found that both a significant rate enhancement and higher yield can be obtained by addition of a tertiary amine to the Paal-Knorr reaction mixture. The quality of the acetonide ester produced by the improved process is at least comparable to that produced by the prior art process.

The catalyst salts of the invention were surprisingly found to work efficiently in a wide range of solvents of varying polarity. The catalyst salts were found to work efficiently in organic solvents, which solvents are important for water removal and solubility of the reactants. Optimal solvent combinations for the improved process are mixtures of MTBE:THF and Hexane:THF. However, solvents may be selected from any one or more of toluene or other aromatic hydrocarbons, THF, or other cyclic and acyclic ethers, ethyl acetate and other esters of alcohols with carboxylic acids, 2-butanone, MIBK and other cyclic and acyclic ketone, methanol and other alcohols, IPA, acetonitrile, DMSO, DMF and other amides, hexane, heptane, and other acyclic and cyclic aliphatic hydrocarbons. Aliphatic nitriles may also be used as solvents. Binary mixtures of the above may also be used such as 1,4-dioxane:MTBE, 1,4-dioxane:Hexane, hexane:acetonitrile, 1,4-dioxane:methylcyclohexane, 1,4-dioxane:cyclohexane, DME:hexane.

The catalyst salt is comprised of a tertiary aromatic or aliphatic amine salt of an organic acid. The acid may be a weak or strong acid.

The weak organic acid having a pKa ≥ 2 and may be selected from but is not limited to any one of pivalic acid, formic acid, acetic acid or isobutyric acid.

A strong acid having a pKa ≤ 2 may also be selected from but is not limited to any one or more of p-toluene sulphonic acid, oxalic acid or trifluoroacetic acid.

The amine of the catalyst may is a tertiary amine. The tertiary amine may be selected from, but is not limited to any one or more of aromatic or aliphatic amines such as, pyridine, *N-*ethylmorpholine, diisopropylethylamine, triethylamine or 1-butylimidazole or N,N-dimethylaniline.

The salt catalyst may be selected from but is not limited to any one or more of pyridinium.pivalate, *n*-ethylmorpholine.pivalate, triethylammonium.acetate, triethylammonium.formate, triethylammonium.p-toluene-sulphonate, triethylammonium.pivalate, triethylammonium.isobutyrate, triethylammoniium.oxalate, triethylammonium.triflouroacetate, 1-butylimidazolium.pivalate, 1-butylimidazolium.isobutyrate, 1-butylimidazolium.oxalate, 1-butylimidazolium.trifluroacetate.

The Paal Knorr condensation reaction results in the formation of water and the presence of water may have a detrimental effect on the overall reaction rate. The salt catalysts of the invention are non-volatile and allow for easy and efficient removal of the water by distillation without removing or affecting the catalyst activity which is important for high yield.

The reaction is carried out in the presence of an organic solvent which also provides for the removal of water formed during the condensation reaction.

The reaction may be carried out in less than 90 hours and the process of the present invention results in a 20% reduction in reaction time over the processes described in Wade et al, Organic Research Process and Development, 1997, 1, 320-324.

The invention will be more clearly understood by the following examples. The catalyst may be prepared in situ as described in the following examples. Alternatively, the catalyst can be prepared by mixing equimolar amounts of the acid and base. The product of this reaction can then be used in the process.

### Example 1: Triethylammonium pivalate as catalyst; hexane: THF as solvent

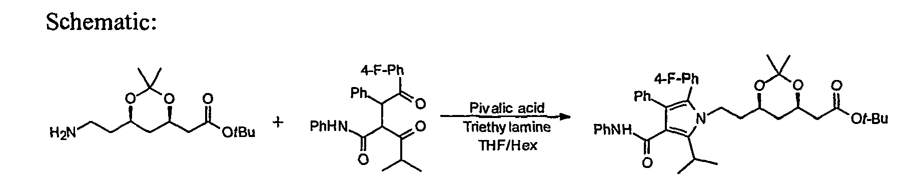

Diketone of atorvastatin (1.09 eq, 84.4g) is added to a solution of 1,1-dimethyl-(4R-*cis*)-6-(2-aminoethyl)-2,2-dimethyl-1,3-dioxane-4-acetate [TBIN] (50.69g, 185.4 mmol) in THF (112.6 g) and hexane (42.7 g) and the mixture warmed to 50°C under an N₂ atmosphere. At 50°C pivalic acid (0.7 eq, 13.3g) is added, followed by triethylamine (0.7 eq., 13.2 g) and the resulting suspension heated at reflux under a nitrogen atmosphere until reaction completion, with concomitant removal of water.

On completion the reaction mixture is cooled to 25°C before addition of MTBE (157 g). The solution is washed with a 5.5 % (w/w) solution of KOH, then a 3.5 % (w/w) of HCl, separated and then the organic layer is vacuum distilled with stirring to a thick oil. At the distillation end-point acetone (100 ml) and IPA (100ml) were added and the mixture was heated to 50°C until a solution is achieved. Water (50ml) was added to the solution and the mixture was allowed to cool to room temperature over two hours, then cooled to 0°C and stirred for 1 hr. The crystals were isolated by filtration and washed with IPA (50ml at 0°C). The product was dried in a vacuum oven maintaining the temperature at <40°C.

### Example 2: Triethylammonium pivalate as catalyst; MTBE: THF as solvent

Diketone of atorvastatin (1.09 eq, 84.4g) is added to a solution of 1,1-dimethyl-(4R-*cis*)-6-(2-aminoethyl)-2,2-dimethyl-1,3-dioxane-4-acetate (50.69g, 185.4 mmol) in THF (57.7 g) and MTBE (99.4 g) and the mixture warmed to 50°C under an N₂ atmosphere. At 50°C pivalic acid (0.7 eq, 13.3g) is added, followed by triethylamine (0.7 eq., 13.2 g) and the resulting suspension heated at reflux under a nitrogen atmosphere until reaction completion, with concomitant removal of water.

On completion the reaction mixture is cooled to 25°C before addition of MTBE (157 g). The solution is washed with a 5.5 % (w/w) solution of KOH (230 ml), then a 3.5 % (w/w) of HCl (250 ml), separated and then the organic layer is vacuum distilled with stirring to a thick oil. At the distillation end-point acetone (100 ml) and IPA (100ml)) were added and the mixture was heated to 50°C until a solution is achieved. Water (50ml) was added to the solution and the mixture was allowed to cool to room temperature over two hours, then cooled to 0°C and stirred for 1 hr. The crystals were isolated by filtration and washed with IPA (50ml at 0°C). The product was dried in a vacuum oven maintaining the temperature at <40°C.

### Example 3: N-ethylmorpholine pivalate as catalyst; Toluene as solvent

Diketone of atorvastatin (1.09 eq, 8.32 g) is added to a solution of 1,1-dimethyl-(4R-*cis*)-6-(2-aminoethyl)-2,2-dimethyl-1,3-dioxane-4-acetate (5.0g, 18.3 mmol) in Toluene (13 g) and the mixture warmed to 50°C under an N₂ atmosphere. At 50°C pivalic acid (0.7 eq, 1.15 g) is added, followed by n-ethylmorpholine (0.7 eq., 1.47 g) and the resulting suspension heated at reflux under a nitrogen atmosphere until reaction completion, with concomitant removal of water.

### Example 4: Diisopropylethylammonium pivalate as catalyst; toluene as solvent

Diketone of atorvastatin (1.09 eq, 8.32 g) is added to a solution of 1,1-dimethyl-(4R-*cis*)-6-(2-aminoethyl)-2,2-dimethyl-1,3-dioxane-4-acetate (5.0g, 18.3 mmol) in Toluene (13 g) and the mixture warmed to 50°C under an N₂ atmosphere. At 50°C pivalic acid (0.7 eq, 1.15 g) is added, followed by diisopropylethylamine (0.7 eq., 1.65 g) and the resulting suspension heated at reflux under a nitrogen atmosphere until reaction completion, with concomitant removal of waster

### Reference Example 5: Zinc acetate as catalyst; MTBE:THF as solvent

Diketone of atorvastatin (1.09 eq, 8.3g) is added to a solution of 1,1-dimethyl-(4R-*cis*)-6-(2-aminoethyl)-2,2-dimethyl-1,3-dioxane-4-acetate (5.0g, 18.5 mmol) in THF (5.7 g) and MTBE (6.7g). Zinc acetate (1.59g, 12.9 mmol) is added and the resulting suspension then heated at reflux under a nitrogen atmosphere until reaction completion, with concomitant removal of water.

### Reference Example 6: Sodium pivalate as catalyst; MTBE:THF as solvent

Diketone of atorvastatin (1.09 eq, 8.32 g) is added to a solution of 1,1-dimethyl-(4R-*cis*)-6-(2-aminoethyl)-2,2-dimethyl-1,3-dioxane-4-acetate (5 g, 18.3 mmol) in THF (5.3 g) and MTBE (6.7g). and the mixture warmed to 50°C under an N₂ atmosphere. At 50°C pivalic acid (0.7 eq, 0.34g) is added, followed by 50% sodium hydroxide (1 g, 12.5 mmol) and the resulting suspension heated at reflux under a nitrogen atmosphere until reaction completion, with concomitant removal of water.

### Reference Example 7: Calcium pivalate as catalyst; MTBE:THF as solvent

Diketone of atorvastatin (1.09 eq, 8.32 g) is added to a solution of 1,1-dimethyl-(4R-*cis*)-6-(2-aminoethyl)-2,2-dimethyl-1,3-dioxane-4-acetate (5 g, 18.3 mmol) in THF (5.3 g) and MTBE (6.7g). and the mixture warmed to 50°C under an N₂ atmosphere. At 50°C pivalic acid (0.7 eq, 1.15g) is added, followed by calcium hydroxide (0.47 g, 6.35mmol)) and the resulting suspension heated at reflux under a nitrogen atmosphere until reaction completion, with concomitant removal of water.

### Reference Example 8: Sodium pivalate as catalyst; Toluene as solvent

Diketone of atorvastatin (1.09 eq, 5.03 g) is added to a solution of 1,1-dimethyl-(4R-*cis*)-6-(2-aminoethyl)-2,2-dimethyl-1,3-dioxane-4-acetate (3 g, 10.9 mmol) in toluene (8.65g) and the mixture warmed to 50°C under an N₂ atmosphere. At 50°C pivalic acid (0.7 eq, 0.69g) is added, followed by 50% sodium hydroxide (0.61 g, 7.6 mmol) and the resulting suspension heated at reflux under a nitrogen atmosphere until reaction completion, with concomitant removal of water.

### Reference Example 9: Triethylammonium hydrochloride as catalyst; MTBE:THF as solvent

Diketone of atorvastatin (1.09 eq, 8.32 g) is added to a solution of 1,1-dimethyl-(4R-*cis*)-6-(2-aminoethyl)-2,2-dimethyl-1,3-dioxane-4-acetate (5 g, 18.3 mmol) in MTBE (6.7g) and THF (5.4 g) and the mixture warmed to 50°C under an N₂ atmosphere. At 50°C triethylamine (0.7 eq, 1.29g) is added, followed by 36% hydrogen chloride (0.7 eq, 1.30 g) and the resulting suspension heated at reflux under a nitrogen atmosphere until reaction completion, with concomitant removal of water.

High yield is critical in this late stage of the process when all the functionality of the active pharmaceutical ingredient, including two chiral centres are essentially in place and this invention thus ensures a major cost benefit. The current process with long reaction times (>100 hours for the Paal-Knorr reaction) is a major bottleneck in throughput and is a significant consumer of energy since continuous heating is required. The preferred embodiment of the invention (amine salt of an organic acid) gives a 3% increase in yield and 20% reduction in reaction time vs. the current process. Because the present invention increases throughput and thus capacity it has the added benefit of saving energy.

The process of the invention gives consistently higher yields and much reduced cycle times for the Paal-Knorr cyclisation reaction. These improvements enable considerably more efficient usage of plant during production of atorvastatin. The invention is particularly useful in factory scale production in which reductions in reaction time and improvements in yield are economically very significant.

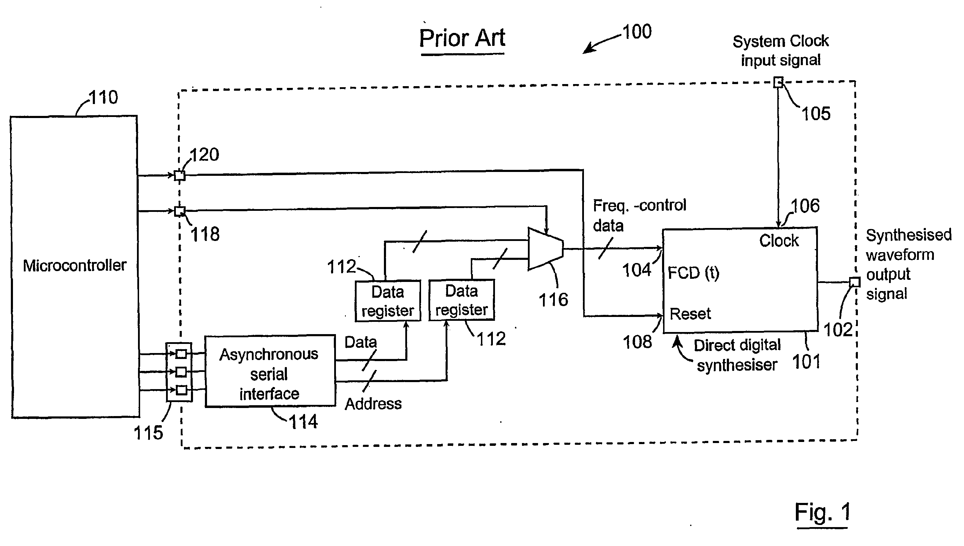

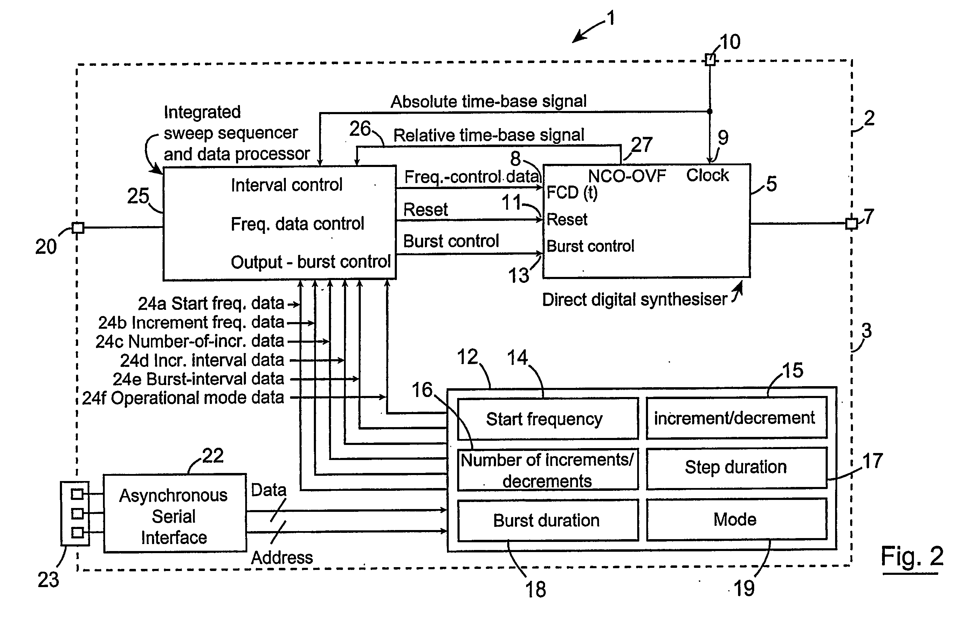

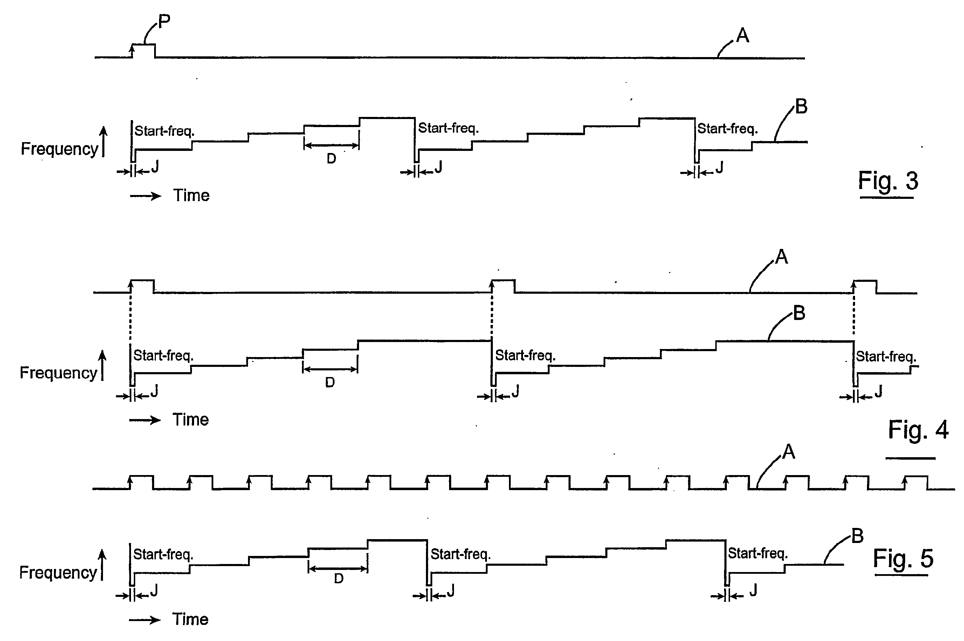

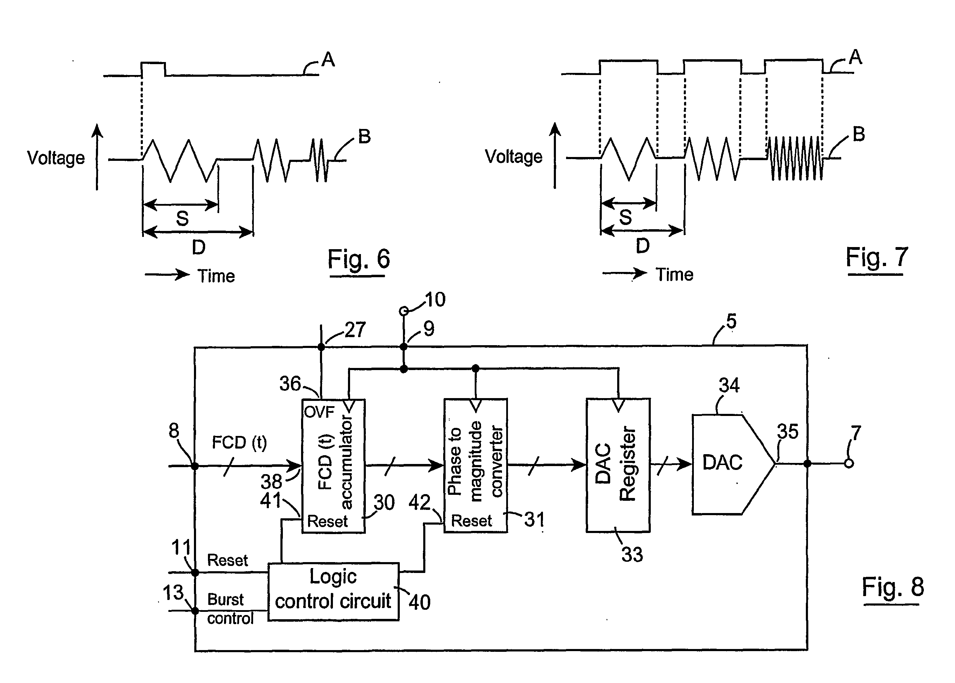

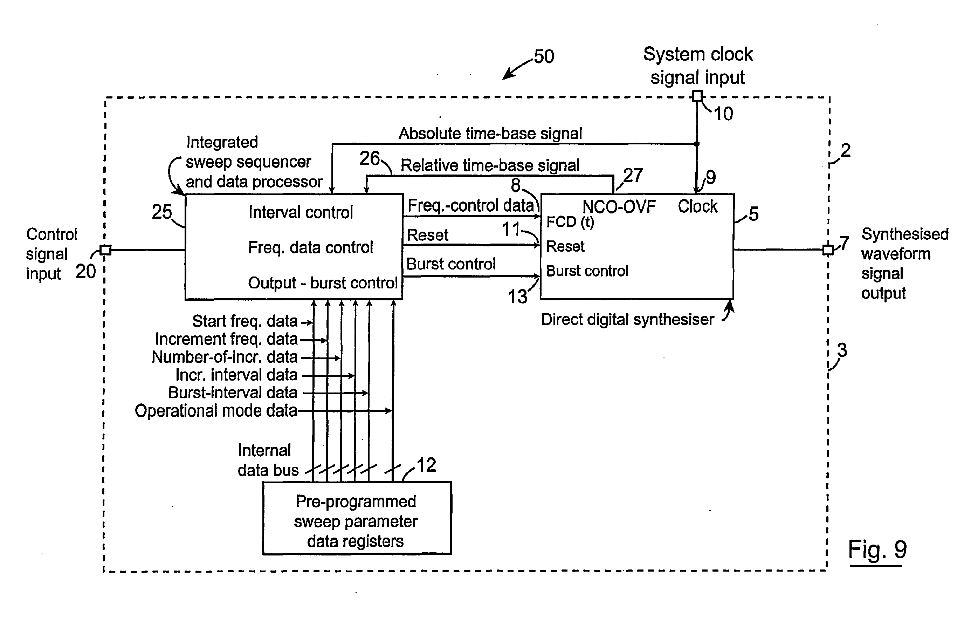

## Claims

1. A process for preparing a compound of formula 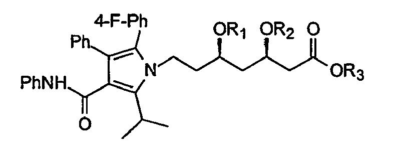 comprising reacting a 1,4-diketone of the formula 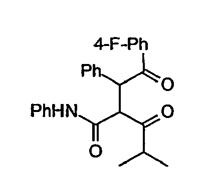 with a primary amine of the formula 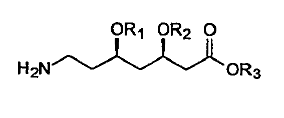 wherein R₁ and R₂ may be the same or different and are selected from any one or more of
H,
C₁-C₆ alkyl which may be straight or branched, substituted or unsubstituted with a halogen group,
C₁-C₆ alkoxy group,
or R₁ and R₂ together represent an alkylidene group of the formula CRₐR_{b} wherein Rₐ and R_{b} may be the same or different and are selected from any one or more of a C₁-C₁₁ alkyl group,
and
R₃ is selected from any one or more of a C₁-C₆ alkyl group,
in the presence of a catalyst, the catalyst comprising a salt wherein the salt comprises a tertiary aromatic or aliphatic amine salt of an organic acid.

2. A process as claimed in claim 1 wherein R₁ and R₂, together represent isopropylidene and R₃ is a tertiary butyl.

3. A process as claimed in claim 1 or 2 wherein the primary amine is 1,1-dimethyl-(4R-*cis*)-6-(2-aminoethyl)-2,2-dimethyl-1,3-dioxane-4-acetate.

4. A process as claimed in any of claims 1 to 3 wherein the organic acid is a weak organic acid.

5. A process as claimed in claim 4 wherein the weak organic acid has a pKa ≥ 2.

6. A process as claimed in claim 4 or 5 wherein the weak organic acid is any carboxylic acid having a C₁-C₆ alkyl group which may be straight or branched.

7. A process as claimed in any of claims 4 to 6 wherein the weak organic acid is selected from any one or more of pivalic acid, formic acid, acetic acid and isobutyric acid.

8. A process as claimed in claim 1 wherein the tertiary amine is selected from any one or more of triethylamine (Et₃N), diisopropylethyl amine (DIPEA), pyridine, 1-butylimidazole, and n-ethylmorpholine.

9. A process as claimed in any of claims 1 to 8 wherein the catalyst salt is selected from any one or more of pyridine.pivalate, n-ethylmorpholine.pivalate, triethylamine.acetate, triethylamine.formate, triethylamine.p-toluene sulphonate, triethyamine, triethylamine.isobutyrate, triethylamine.oxalate, triethylamine.triflouroacetate, 1-butylimidazole.pivalate, 1-butylimidazole.isobutyrate, 1-butylimidazole.oxalate, and 1-butylimidazole.trifluroacetate.

10. A process as claimed in any of any of claims 1 to 9 wherein the reaction is carried out in the presence of an inert organic solvent.

11. A process as claimed in claim 10 wherein the inert organic solvent is selected from Hexane:THF, MTBE:THF and toluene.

12. A process as claimed in any of claims 1 to 11 wherein the reaction is carried out at a pH greater than about pH 7.

13. A process as claimed in any of claims 1 to 12 wherein the water formed during the reaction is removed azeotropically.

14. A process as claimed in any of claims 1 to 13 wherein the reaction is carried out at a temperature of between 40 and 120°C.

## Patentansprüche

1. Verfahren zur Herstellung einer Verbindung der Formel 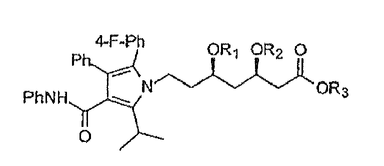 umfassend das Umsetzen eines 1,4-Diketons der Formel 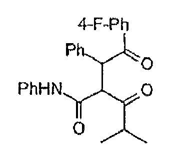 mit einem primären Amin der Formel 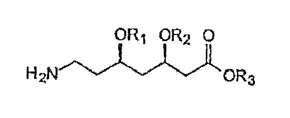 worin R₁ und R₂ gleich oder verschieden sein können und ausgewählt sind aus einem oder mehreren von
H,
C₁-C₆-Alkyl, das geradkettig oder verzweigt, substituiert oder unsubstituiert mit einer Halogengruppe sein kann, C₁-C₆-Alkoxygruppe,
oder R₁ und R₂ stehen zusammen für eine Alkylidengruppe der Formel CRₐR_{b}, worin Rₐ und R_{b} gleich oder verschieden sein können und ausgewählt sind aus einem oder mehreren von einer C₁-C₁₁-Alkylgruppe,
und
R₃ ausgewählt ist aus einem oder mehreren von einer C₁-C₆-Alkylgruppe,
in der Gegenwart eines Katalysators, wobei der Katalysator ein Salz umfasst, wobei das Salz ein tertiäres aromatisches oder aliphatisches Aminsalz einer organischen Säure umfasst.

2. Verfahren gemäß Anspruch 1, wobei R₁ und R₂ zusammen für Isopropyliden stehen und R₃ tertiäres Butyl ist.

3. Verfahren nach Anspruch 1 oder 2, wobei das primäre Amin 1,1-Dimethyl-(4R-*cis*)-6-(2-aminoethyl)-2,2-dimethyl-1,3-dioxan-4-acetat ist.

4. Verfahren nach einem der Ansprüche 1 bis 3, wobei die organische Säure eine schwache organische Säure ist.

5. Verfahren nach Anspruch 4, wobei die schwache organische Säure einen pKa ≥ 2 besitzt.

6. Verfahren nach Anspruch 4 oder 5, wobei die schwache organische Säure eine Carbonsäure mit einer C₁-C₆-Alkylgruppe ist, die geradekettig oder verzweigt sein kann.

7. Verfahren nach einem der Ansprüche 4 bis 6, wobei die schwache organische Säure ausgewählt ist aus einer oder mehreren von Pivalinsäure, Ameisensäure, Essigsäure und IsoButtersäure.

8. Verfahren nach Anspruch 1, wobei das tertiäre Amin augewählt ist aus einem oder mehreren von Triethylamin (Et₃N), Diisopropylethylamin (DIPEA), Pyridin, 1-Butylimidazol und n-Ethylmorpholin.

9. Verfahren nach einem der Ansprüche 1 bis 8, wobei das Katalysatorsalz ausgewählt ist aus einem oder mehreren von Pyridin.Pivalat, n-Ethylmorpholin.Pivalat, Triethylamin.Acetat, Triethylamin.Formiat, Triethylamin.p-Toluolsulfonat, Triethylamin, Triethylamin.Isobutyrat, Triethylamin.oxalat, Triethylamin.Trifluoracetat, 1-Butylimidazol.Pivalat, 1-Butylimidazol.Isobutyrat, 1-Butylimidazol.Oxalat und 1-Butylimidazol.Trifluoracetat.

10. Verfahren nach einem der Ansprüche 1 bis 9, wobei die Reaktion durchgeführt wird in Gegenwart eines inerten organischen Lösungsmittels.

11. Verfahren nach Anspruch 10, wobei das inerte organische Lösungsmittel ausgewählt ist aus Hexan:THF, MTBE:THF und Toluol.

12. Verfahren nach einem der Ansprüche 1 bis 11, wobei die Reaktion durchgeführt wird bei einem pH-Wert größer als etwa pH 7.

13. Verfahren nach einem der Ansprüche 1 bis 12, wobei das während der Reaktion gebildete Wasser azeotroph entfernt wird.

14. Verfahren nach einem der Ansprüche 1 bis 13, wobei die Reaktion durchgeführt wird bei einer Temperatur zwischen 40 und 120°C.

## Revendications

1. Procédé de préparation d'un composé de formule 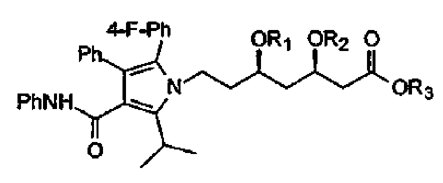 comprenant la réaction d'une 1,4-dicétone de formule 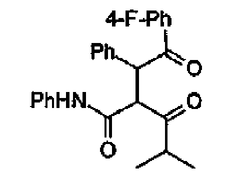 avec une amine primaire de formule 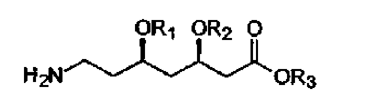 où R₁ et R₂ peuvent être identiques ou différents et sont sélectionnés de manière quelconque parmi un ou plusieurs des éléments suivants :
H,
alkyle en C₁-C₆ qui peut être droit ou ramifié, substitué ou non-substitué par un groupe halogéno,
un groupe alcoxy en C₁-C₆,
ou R₁ et R₂ représentent ensemble un groupe alkylidène de formule CRₐR_{b}, dans lequel Rₐ et R_{b} peuvent être identiques ou différents et sont sélectionnés de manière quelconque parmi un ou plusieurs groupe(s) alkyle en C₁-C₁₁,
et
R₃ est sélectionné de manière quelconque parmi un ou plusieurs groupe(s) alkyle en C₁-C₆,
en présence d'un catalyseur, le catalyseur étant un sel, ce sel étant un sel d'amine aromatique ou aliphatique tertiaire d'un acide organique.

2. Procédé tel que revendiqué dans la revendication 1, dans lequel R₁ et R₂ représentent ensemble un groupe isopropylidène et R₃ est un butyle tertiaire.

3. Procédé tel que revendiqué dans la revendication 1 ou 2, dans lequel l'amine primaire est le 1,1-diméthyl-(4R-*cis*)-6-(2-aminoéthyl)-2,2-diméthyl-1,3-dioxane-4-acétate.

4. Procédé tel que revendiqué dans l'une quelconque des revendications 1 à 3, dans lequel l'acide organique est un acide organique faible.

5. Procédé tel que revendiqué dans la revendication 4, dans lequel l'acide organique faible a une pKa ≥ 2.

6. Procédé tel que revendiqué dans la revendication 4 ou 5, dans lequel l'acide organique faible est tout acide carboxylique ayant un groupe alkyle en C₁-C₆ qui peut être droit ou ramifié.

7. Procédé tel que revendiqué dans l'une quelconque des revendications 4 à 6, dans lequel l'acide organique faible est sélectionné de manière quelconque parmi un ou plusieurs des éléments suivants : l'acide pivalique, l'acide formique, l'acide acétique et l'acide isobutyrique.

8. Procédé tel que revendiqué dans la revendication 1, dans lequel l'amine tertiaire est sélectionnée de manière quelconque parmi un ou plusieurs des éléments suivants : la triéthylamine (Et₃N), la diisopropyléthylamine (DIPEA), la pyridine, le 1-butylimidazole et la n-éthylmorpholine.

9. Procédé tel que revendiqué dans l'une quelconque des revendications 1 à 8, dans lequel le sel catalyseur est sélectionné de manière quelconque parmi un ou plusieurs des éléments suivants : le pivalate de pyridine, le pivalate de n-éthylmorpholine, l'acétate de triéthylamine, le formiate de triéthylamine, le p-toluène-sulfonate de triéthylamine, la triéthylamine, l'isobutyrate de triéthylamine, l'oxalate de triéthylamine, le trifluoroacétate de triéthylamine, le pivalate de 1-butylimidazole, l'isobutyrate de 1-butylimidazole, l'oxalate de 1-butylimidazole et le trifluoroacétate de 1-butylimidazole.

10. Procédé tel que revendiqué dans l'une quelconque des revendications 1 à 9, dans lequel la réaction est mise en oeuvre en présence d'un solvant organique inerte.

11. Procédé tel que revendiqué dans la revendication 10, dans lequel le solvant organique inerte est sélectionné parmi un mélange hexane:THF, MTBE:THF et le toluène.

12. Procédé tel que revendiqué dans l'une quelconque des revendications 1 à 11, dans lequel la réaction est mise en oeuvre à un pH supérieur à environ pH 7.

13. Procédé tel que revendiqué dans l'une quelconque des revendications 1 à 12, dans lequel l'eau formée pendant la réaction est éliminée de manière azéotropique.

14. Procédé tel que revendiqué dans l'une quelconque des revendications 1 à 13, dans lequel la réaction est mise en oeuvre à une température comprise entre 40 et 120°C.
